(19) Europäisches Patentamt
European Patent Office
Office européen des brevets

(11) Veröffentlichungsnummer: **0 201 094**
**A2**

# (12) EUROPÄISCHE PATENTANMELDUNG

(21) Anmeldenummer: **86106232.1**

(22) Anmeldetag: **07.05.86**

(51) Int. Cl.⁴: **C07D 495/04**, A61K 31/435, //(C07D495/04,333:00,235:00)

(30) Priorität: **07.05.85 AT 1360/85**

(43) Veröffentlichungstag der Anmeldung: **17.12.86 Patentblatt 86/46**

(84) Benannte Vertragsstaaten: **AT BE CH DE FR GB IT LI LU NL SE**

(71) Anmelder: **CHEMIE LINZ AKTIENGESELLSCHAFT**
**St. Peter-Strasse 25**
**A-4020 Linz(AT)**
Anmelder: **Lentia Gesellschaft mit beschränkter Haftung**
**Arabellastrasse 4 Postfach 81 05 08**
**D-8000 München 81(DE)**

(72) Erfinder: **Binder, Dieter, Dr.**
**Sieveringerstrasse 207**
**A-1190 Wien(AT)**
Erfinder: **Rovenszky, Franz, Dipl.-Ing. Dr.**
**Lagerhausstrasse 5/8**
**A2460 Bruck a.d. Leitha(AT)**

## (54) Neue Derivate von Thieno[2,3-d]-imidazolen und Verfahren zu ihrer Herstellung.

(57) Die Erfindung betrifft neue Derivate von Thieno-(2,3-d)imidazolen der allgemeinen Formel I des Formelblattes,

in der

$R_1$ Wasserstoff, niederes Alkyl, Chlor, Brom, Acetyl, Cyclopropylcarbonyl, Methoxycarbonyl, Ethoxycarbonyl

$R_2$ Wasserstoff oder niederes Alkyl

$R_3$, $R_5$ und $R_6$ unabhängig voneinander Wasserstoff oder niederes Alkyl,

$R_4$ Wasserstoff oder niederes Alkoxy und

n O oder 1 bedeuten, und deren pharmazeutisch verträgliche Säureadditionssalze, ein Verfahren zu ihrer Herstellung und pharmazeutische Präparate, die diese Verbindungen enthalten. Die neuen Verbindungen bewirken eine Blockade der ($H^+$ + $K^+$) ATPase und können als Wirkstoffe für Arzneimittel zur Behandlung und Prophylaxe von Krankheiten, die durch eine erhöhte Magensekretion hervorgerufen werden, verwendet werden.

EP 0 201 094 A2

# Neue Derivate von Thieno(2,3-d)imidazolen und Verfahren zu.ihrer Herstellung

Die Erfindung betrifft neue Derivate von Thieno(2,3-d)imidazolen der allgemeinen Formel I des Formelblattes,

in der

$R_1$ Wasserstoff, niederes Alkyl, Chlor, Brom, Acetyl, Cyclopropylcarbonyl, Methoxycarbonyl oder Ethoxycarbonyl,

$R_2$ Wasserstoff oder niederes Alkyl,

$R_3$, $R_5$ und $R_6$ unabhängig voneinander Wasserstoff oder niederes Alkyl,

$R_4$ Wasserstoff oder niederes Alkoxy und

n 0 oder 1 bedeuten

und deren pharmazeutisch verträgliche Säureadditionssalze, Verfahren zu ihrer Herstellung, pharmazeutische Präparate, die diese Verbindungen enthalten, sowie ihre Verwendung.

Der in dieser Beschreibung verwendete Ausdruck "niederes Alkyl" bezeichnet geradkettige oder verzweigte gesättigte Kohlenwasserstoffgruppen mit 1 -4 Kohlenstoffatomen, wie z.B. Methyl, Ethyl, Propyl, Isopropyl, Butyl, tert.-Butyl. Der Ausdruck "niederes Alkoxy" bezieht sich auf Hydrocarbonoxygruppen mit 1 -4 Kohlenstoffatomen, z.B. Methoxy, Ethoxy, Propyloxy, Isopropyloxy, Butyloxy, tert.-Butyloxy.

In einer bevorzugten Gruppe von Verbindungen der Formel I bedeutet $R_1$ Wasserstoff, Acetyl, Methoxycarbonyl oder Ethoxycarbonyl, wobei Wasserstoff oder Acetyl besonders bevorzugt sind. $R_2$ ist bevorzugt Wasserstoff. $R_3$, $R_5$ und $R_6$ stellen bevorzugt Wasserstoff oder Methyl dar. $R_4$ steht bevorzugt für Wasserstoff oder Methoxy.

Eine besonders bevorzugte Gruppe innerhalb der Verbindungen der allgemeinen Formel I ist jene, in der n die Zahl 1 bedeutet.

Die Thieno(2,3-d)imidazolderivate der allgemeinen Formel I und ihre Salze können erfindungsgemäß dadurch hergestellt werden, daß man

a) eine Verbindung der allgemeinen Formel II des Formelblattes,

worin $R_1$ und $R_2$ die in Formel I angegebene Bedeutung haben, mit einer Verbindung der allgemeinen Formel III des Formelblattes,

worin

X für Chlor oder Brom steht und

$R_3$, $R_4$, $R_5$ und $R_6$ die in Formel I angegebenen Bedeutung haben, in Gegenwart von mindestens 2 Äquivalenten einer starken Base umsetzt, worauf man

b) gegebenenfalls die so erhaltenen Verbindungen der allgemeinen Formel I, worin n = 0 bedeutet, mit äquivalenten Mengen einer organischen Persäure oder Wasserstoffperoxid zu einer Verbindung der allgemeinen Formel I umsetzt, in der n die Zahl 1 bedeutet, und

c) erwünschtenfalls eine in Stufe a) oder b) erhaltene Verbindung der allgemeinen Formel I in ein pharmazeutisch verträgliches Säureadditionssalz überführt.

Die Umsetzung nach Verfahrensschritt a) wird vorteilhafterweise so durchgeführt, daß man die Verbindungen der Formel II und III in einem inerten organischem Lösungsmittel, beispielsweise einem niedersiedenden aliphatischen Alkohol, wie Methanol, Ethanol, Isopropanol und dergleichen, vorzugsweise in Methanol, suspendiert, wobei man aus Gründen der besseren Aufarbeitbarkeit die Verbindung der Formel II zweckmäßigerweise im Überschuß einsetzt und dann mindestens 2 Äquivalente einer starken Base, vorzugsweise NaOH oder KOH, gelöst in etwas Wasser, bei Raumtemperatur zutropfen läßt. Die Reaktionszeit beträgt 2 -4 Stunden.

Gemäß Verfahrensschritt b) können die Sulfoxidverbindungen, bei denen n in der allgemeinen Formel I die Zahl 1 bedeutet, ausgehend von den nach Verfahrensschritt a) erhaltenen Sulfidverbindungen mit der Bedeutung für n = 0 durch partielle Oxidation mit geeigneten Oxidationsmitteln erhalten werden. Als solche Oxidationsmittel werden vorzugsweise äquivalente Mengen einer organischen Persäure wie Peressigsäure, Perbenzoesäure oder m-Chlorperbenzoesäure, in einem reaktionsinerten Lösungsmittel, z.B. Methylenchlorid oder Chloroform, zweckmäßigerweise bei Temperaturen zwischen -5° C und -50° C, oder äquivalente Mengen 30 proz. $H_2O_2$ in Eisessig vorteilhafterweise bei Raumtemperatur verwendet.

Die Verbindungen der allgemeinen Formel I unterliegen der Tautomerie und können daher auch in allen zu Formel I tautomeren Formen vorliegen.

Die Ausgangsverbindungen der allgemeinen Formel III sind bekannt. Die für das Verfahren verwendeten Ausgangsverbindungen der allgemeinen Formel II sind entweder bekannt oder können ausgehend von bekannten Produkten in an sich bekannter Weise hergestellt werden. Insbesondere können sie gemäß den folgenden Reaktionsschemata und gemäß den spezifischen Angaben in den Beispielen synthetisiert werden. Die in den Reaktionsschemata angegebenen Startverbindungen der Formeln IV a, IV b, V und VI sind literaturbekannt. Reaktionsschema I bezieht sich auf die Herstellung von Verbindungen der Formel II, in denen $R_1$ Wasserstoff, niederes Alkyl, Chlor oder Brom und $R_2$ Wasserstoff oder niederes Alkyl bedeuten und Reaktionsschema II auf die Herstellung von Verbindungen der Formel II in denen $R_1$ Acetyl, Cyclopropylcarbonyl, Methoxycarbonyl oder Ethoxycarbonyl und $R_2$ wiederum Wasserstoff oder niederes Alkyl bedeuten.

Reaktionsschema I

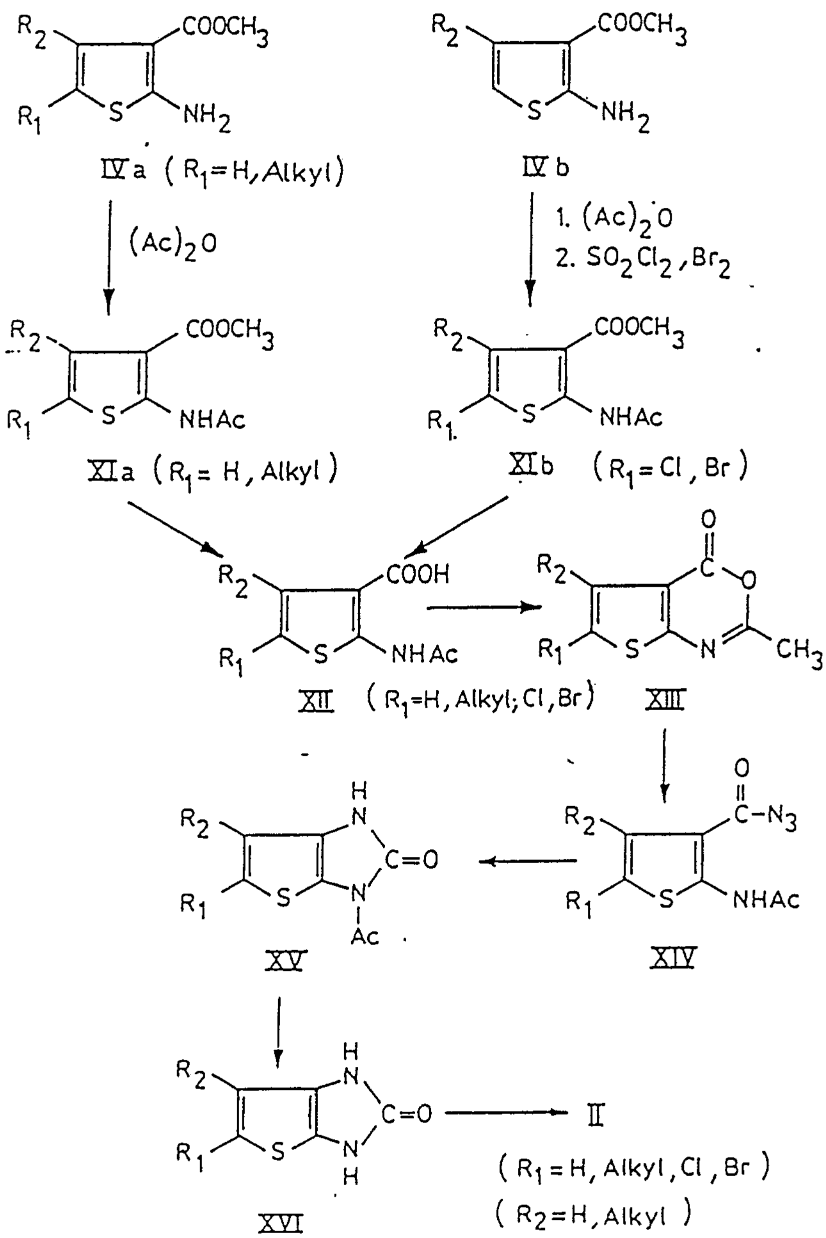

Reaktionsschema II

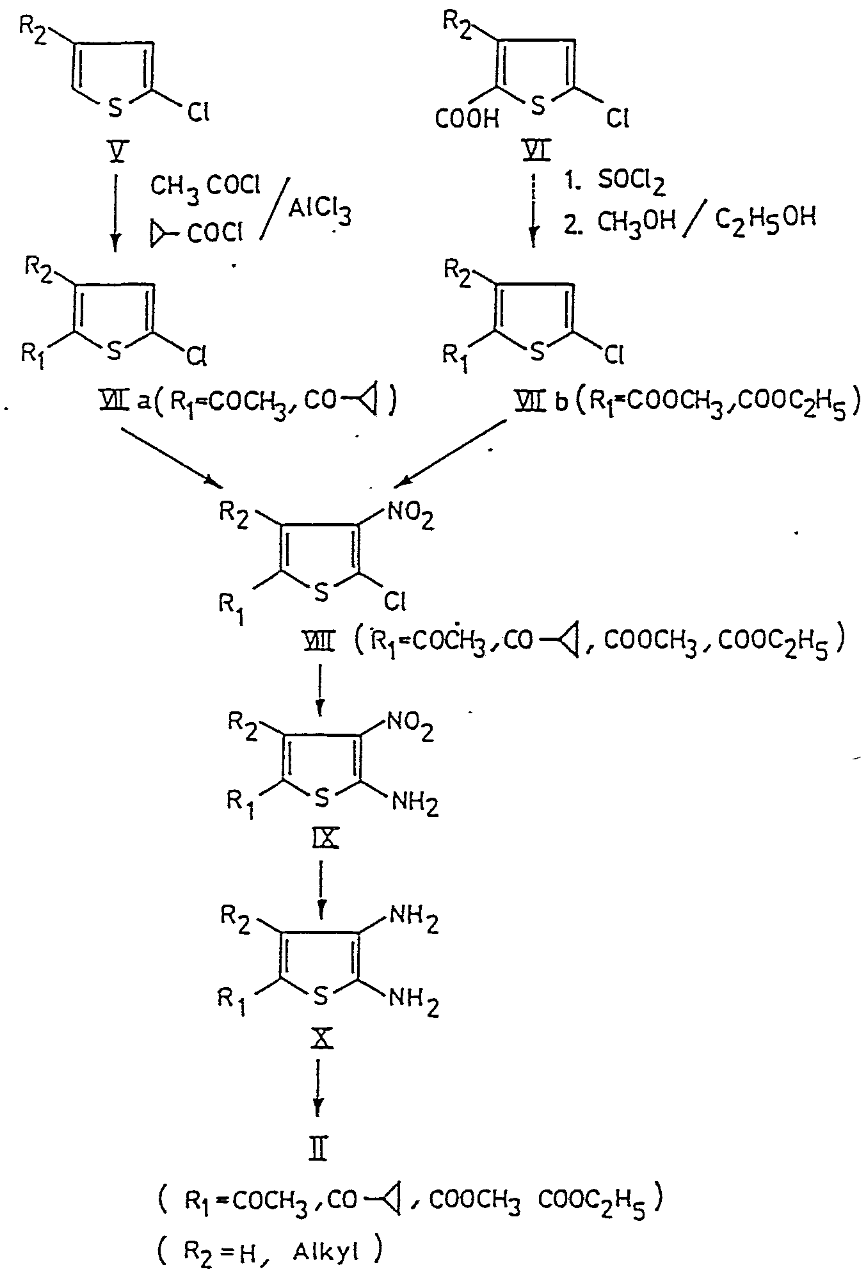


Die Verbindungen der allgemeinen Formel I haben stark basische Eigenschaften. Man kann sie daher gemäß Verfahrensschritt c) leicht in kristalline, pharmazeutisch verträgliche Säureadditionssalze überführen, die sich wie z.B. die Hydrochloride gut durch Umkristallisieren reinigen lassen. Dazu löst man die rohe Base zweckmäßigerweise in einem geeigneten Lösungsmittel, z.B. in einem niederen Alkohol, gibt eine äquivalente Menge Protonensäure dazu, dampft im Vakuum das Lösungsmittel ab und kristallisiert den Rückstand aus Methanol oder Ethanol, gegebenenfalls unter Zusatz von Ether, um.

Geeignete Beispiele für derartige pharmazeutisch verträgliche Salze sind neben dem Salz der Salzsäure beispielsweise die Salze der Schwefelsäure, der Salpetersäure, der Phosphorsäure, von Sulfonsäuren, der Benzoesäure, der Maleinsäure, der Weinsäure oder der Zitronensäure.

Die Verbindungen der allgemeinen Formel I und ihre pharmazeutisch verträglichen Säureadditionssalze zeigen im Tierversuch wertvolle pharmakologische Eigenschaften. Insbesondere bewirken sie eine Blockade der ($H^+$ + $K^+$) - ATPase und können daher beispielsweise zur Behandlung oder Prophylaxe von Magen-und Zwölffingerdarmgeschwüren und anderen durch erhöhte Magensekretion verursachte Erkrankungen in der Humanmedizin eingesetzt werden. Zur Untersuchung der pharmakologischen Eigenschaften wurde folgende Testmethode verwendet:

Die Substanzen wurden in 20 %-igem Dimethylsulfoxid gelöst und wachen weiblichen Ratten - (Stamm: Charles River CD) in ansteigenden Dosen von 125, 250 und 500 µmol/kg mit dem einheitlichen Dosierungsvolumen von 20 ml/kg oral appliziert. 60 Minuten später wurden die Tiere einer Pylorusligatur unterzogen. 4 Stunden nach Setzen der Pylorusligatur wurde die Säurekonzentration und der Gesamtsäuregehalt im Magenvolumen der Tiere bestimmt. Die Kontrollgruppe erhielt 20 ml/kg Dimethylsulfoxid bzw. 20 ml/kg destilliertes Wasser.

In diesem Standardtest bewirken Verbindungen der allgemeinen Formel I, wie beispielweise 5-Acetyl-2-((4-methoxy-3,5-dimethyl-2-pyridyl)-methylsulfinyl)-3H-thieno(2,3-d)imidazol (Verbindung A) oder 2-((4-Methoxy-2-pyridyl)methylsulfinyl-3H-thieno(2,3-d)-imidazol (Verbindung B), eine signifikante, dosisabhängige Hemmung der Magensekretion - (Tabellen I und II).

Tabelle I:

Magensaftsekretion bei Ratten mit Pylorusligatur nach Applikation von Verbindungen der allgemeinen Formel I

| | Menge (ml) | Säurekonzentration (mÄq/ml) | Säure gesamt (mÄq) |
|---|---|---|---|
| Wasser (Kontrolle) | 4.7±0.7 | 0.12±0.005 | 0.56±0.09 |
| DMSO (Träger) | 3.8±0.5 | 0.08±0.008 | 0.34±0.07 |
| Verbindung A | | | |
| 125 µmol/kg | 4.7±0.4 | 0.08±0.008 | 0.37±0.06 |
| 250 µmol/kg | 3.4±0.3 | 0.04±0.006 | 0.16±0.03 |
| 500 µmol/kg | 3.1±0.4 | 0.04±0.005 | 0.13±0.03 |

Tabelle II:

Magensaftsekretion bei Ratten mit Pylorusligatur nach Applikation von Verbindungen der allgemeinen Formel I

| | Menge (ml) | Säurekonzentration (mÄq/ml) | Säure gesamt (mÄq) |
|---|---|---|---|
| Wasser (Kontrolle) | 4.9±0.6 | 0.13±0.003 | 0.62±0.08 |
| DMSO (Träger) | 5.1±0.4 | 0.10±0.006 | 0.50±0.05 |
| Verbindung B | | | |
| 125 µmol/kg | 4.5±0.6 | 0.08±0.007 | 0.37±0.07 |
| 250 µmol/kg | 5.0±0.6 | 0.05±0.004 | 0.27±0.05 |
| 500 µmol/kg | 4.5±0.4 | 0.03±0.004 | 0.14±0.02 |

Die Verbindungen der allgemeinen Formel I sowie deren Salze können als Heilmittel, z.B. in Form pharmazeutischer Präparate Verwendung finden, welche die erfindungsgemäßen Verbindungen in Mischung mit einem für die enterale oder parenterale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, Polyalkylenglykole, Vaseline oder dergleichen enthalten. Die pharmazeutischen Präparate können in fester Form z.B. als Tabletten, Dragees, Suppositorien, Kapseln, oder in flüssiger Form z.B. als Lösungen, Suspensionen oder Emulsionen vorliegen. Gegebenenfalls sind sie sterilisiert und/oder enthalten Hilfsstoffe, wie Konservierungs-, Stabilisierungs-oder Emulgiermittel, Salze zur Veränderung des osmotischen Druckes oder Puffer. Sie können auch in Kombination mit anderen therapeutisch wertvollen Stoffen verabreicht werden.

Die nachfolgenden Beispiele erläutern die Erfindung.

Beispiel 1:

5-Methyl-2-(2-pyridylmethylthio)-3H-thieno(2,3-d)-imidazol

(Formel I: $R_1 = CH_3$, $R_2$ -$R_6$ = H, n = 0)

1,56 g (9,162 mmol) 1,3-Dihydro-5-methyl-thieno(2,3-d)imidazol-2-thion (Formel II: $R_1 = CH_3$, $R_2$ = H) und 1,33 g (8,108 mmol) 2-Chlormethylpyridin, Hydrochlorid werden in 40 ml Methanol suspendiert und unter Rühren 8,64 ml 2n NaOH - (17,28 mmol) langsam in 20 Minuten zugetropft. Dabei steigt die Temperatur bis auf 29° C und es bildet sich eine klare Lösung, aus der nach ca. 30 Minuten bereits das Produkt ausfällt. Es wird noch 2,5 Stunden bei Raumtemperatur gerührt, anschließend das Lösungsmittel im Vakuum weitgehend entfernt, der Rückstand in 30 ml Wasser aufgenommen und durch Zugabe von 0,5 ml Eisessig ein pH von 4,5 eingestellt. Es wird viermal mit je 25 ml Chloroform ausgeschüttelt, die vereinigten organischen Phasen über Natriumsulfat getrocknet und eingedampft. Das Rohprodukt (2,0 g, 94 % d.Th.) wird aus Acetonitril umkristallisiert.

Ausbeute: 1,56 g farblose Kristalle (73,6 % d.Th.)

Fp = 171 -172° C ($CH_3CN$)

Das Ausgangsmaterial kann wie folgt hergestellt werden:

2-Acetylamino-5-methyl-3-thiophencarbonsäuremethylester

(XIa: $R_1 = CH_3$, $R_2$ = H)

284,0 g (1,659 mol) 2-Amino-5-methyl-3-thiophencarbonsäuremethylester (IVa, literaturbekannt) werden in 600 ml Essigsäureanhydrid portionsweise eingerührt, wobei die Temperatur durch zwischenzeitliche Eiskühlung zwischen 18° und 25° C

gehalten wird. Nach 2,5 Stunden Rühren wird im Vakuum eingedampft und der bräunliche kristalline Rückstand aus Diisopropylether umkristallisiert.

Ausbeute: 277,5 g gelbliche Kristalle (78,5 % d.Th.)

Fp = 100 -101° C (Diisopropylether)

2-Acetylamino-5-methyl-3-thiophencarbonsäure

(XII: $R_1$ = $CH_3$, $R_2$ = H)

200,0 g (0,938 mol) 2-Acetylamino-5-methyl-3-thiophencarbonsäuremethylester werden in 1400 ml Methanol bei 50° C eingerührt. Unter mechanischem Rühren wird in der Siedehitze eine Lösung von 37,6 g (0,940 mmol) NaOH in 970 ml Wasser innerhalb von 2,5 Stunden zugetropft. Es wird noch 30 Minuten unter Rückfluß erhitzt und die Lösung nach dem Abkühlen weitgehend eingedampft. Der verbleibende Rückstand wird in 1800 ml Wasser und 200 ml gesättigter Natriumhydrogencarbonatlösung aufgenommen und dreimal mit je 250 ml Methylenchlorid extrahiert. Die wäßrige Phase wird mit konz. Salzsäure auf pH = 1 angesäuert, der ausgefallene Niederschlag abgesaugt, dreimal mit Wasser gewaschen und 3 Stunden im Umlufttrockenschrank bei 70° C getrocknet.

Ausbeute: 121,8 g farblose Kristalle (65,2 % d.Th.)

Fp = 197 -198° C (MeOH)

3,6-Dimethyl-4H-thieno(2,3-d)-(1,3)-oxazin-4-on

(XIII: $R_1$ = $CH_3$, $R_2$ = H)

170,0 g (0,853 mol) 2-Acetylamino-5-methyl-3-thiophencarbonsäure (XII) werden in 800 ml Acetanhydrid suspendiert und 2,5 Stunden unter Rückfluß erhitzt. Anschließend wird die Lösung abgekühlt und im Vakuum vollständig eingedampft. Der verbleibende Rückstand wird aus Tetrachlorkohlenstoff umkristallisiert.

Ausbeute: 128,0 g farblose Kristalle (82,8 % d.Th.)

Fp = 140° C (Diisopropylether)

2-Acetylamino-5-methyl-3-thiophencarbonsäureazid

(XIV: $R_1$ = $CH_3$, $R_2$ = H)

72,0 g (0,397 mol) 3,6-Dimethyl-4H-thieno (2,3-d)-(1,3)-oxazin-4-on werden in 1100 ml Dioxan gelöst und mit einer Lösung von 51,7 g (0,795 mol) Natriumazid in 170 ml Wasser unterschichtet. Es wird 50 Stunden bei Raumtemperatur langsam gerührt und in Abständen von 8 -12 Stunden insgesamt 23,8 ml (0,397 mol) Eisessig in 4 Portionen zugesetzt.

Anschließend werden die Phasen getrennt und die organische Phase bei einer Badtemperatur von 40° C eingedampft. Das verbleibende dunkle Öl wird in 600 ml Methylenchlorid aufgenommen und mit der wäßrigen Phase und 150 ml gesättigter Natriumbicarbonatlösung vereinigt. Die Phasen werden getrennt und die wäßrige noch zweimal mit je 200 ml Methylenchlorid ausgeschütttelt. Die vereinigten organischen Extrakte werden über Natriumsulfat getrocknet und eingedampft.

Ausbeute: 72,0 g gelbe Kristalle (80,8 % d.Th.)

Das Rohprodukt kann aus Methanol umkristallisiert werden.

Fp = 78 -79° C (MeOH)

3-Acetyl-1,3-dihydro-5-methyl-thieno(2,3-d)-imidazol-2-on

(XV: $R_1$ = $CH_3$, R = H)

16,3 g (72,7 mmol) 2-Acetylamino-5-methyl-3-thiophencarbonsäureazid werden in 250 ml absol. Dioxan gelöst und 4,5 Stunden bei 100° C erhitzt. Die dunkle Lösung wird über Nacht abkühlen gelassen, der ausgefallene Niederschlag abgesaugt, die Mutterlauge auf das halbe Volumen eingeengt, auskristallisieren gelassen und neuerlich abgesaugt.

Ausbeute: 9,92 g farblose Kristalle (69,6 % d.Th.)

Fp = ca. 200° C Zers. (Dioxan)

1,3-Dihydro-5-methyl-thieno(2,3-d)imidazol-2-on

(XVI: $R_1$ = $CH_3$, $R_2$ = H)

34,7 g (0,177 mol) 2-Acetyl-1,3-dihydro-5-methyl-thieno(2,3-d)-imidazol-2-on werden bei Raumtemperatur in 420 ml 2n NaOH eingetragen und nach vollständiger Auflösung noch 15 Minuten gerührt. Die tiefrote Lösung wird mit konz. HCl tropfenweise auf pH = 2,5 angesäuert, in einem Eisbad kurz gekühlt, der ausgefallene Niederschlag abgesaugt und zweimal mit kaltem Wasser gewaschen. Die Kristalle werden bei 70° C/20 mbar getrocknet.

Ausbeute: 26,5 g farblose Kristalle (97,2 % d.Th.)

Fp = 275 -278° C Zers.

1,3-Dihydro-5-methyl-thieno(2,3-d)imidazol-2-thion

(II: $R_1$ = $CH_3$, $R_2$ = H)

Zu 700 ml abs. Pyridin werden 25,0 g (0,162 mol) 1,3-Dihydro-5-methyl-thieno(2,3-d)imidazol-2-on und 27,0 g (0,122 mol) $P_2S_5$ bei Raumtemperatur unter mechanischem Rühren hinzugegeben. Die Suspension wird unter Stickstoff in einem Ölbad erwärmt, wobei bei ca. 100° C eine klare rote Lösung entsteht. Es wird 3 Stunden unter Rückfluß erhitzt.

Anschließend wird abgekühlt und das Lösungsmittel im Vakuum abdestilliert. Der dunkle zäh-ölige Rückstand wird in 300 ml 2n NaOH gelöst und diese Lösung langsam in eine heftig gerührte Mischung aus 1100 ml Ethylacetat und 400 ml 3n HCl gegossen. Es wird noch 10 Minuten gerührt, über ein Filtrierhilfsmittel abgesaugt und die Phasen getrennt. Die wäßrige Phase wird noch viermal mit insgesamt 800 ml Ethylacetat ausgeschüttelt. Die vereinigten organischen Phasen werden über Natriumsulfat/Aktivkohle getrocknet und eingedampft. Der dunkle Rückstand (12,4 g; 45 % d.Th.) wird in 90 ml 2n NaOH gelöst, mit Aktivkohle filtriert und die klare rote Lösung mit konz. HCl auf pH = 2 angesäuert. Die ausgefallenen Kristalle werden abgesaugt, zweimal mit Wasser gewaschen und im Vakuum bei 60° C getrocknet.

Ausbeute: 9,8 g hellbraune Kristalle (35,5 % d.Th.)

Fp = größer als 330° C, kontinuierliche Zersetzung.

Beispiel 2:

5-Methyl-2-((4-methoxy-2-pyridyl)methylthio)-3H-thieno(2,3-d)imidazol

(I: $R_1$ = $CH_3$, $R_2$, $R_3$, $R_5$ und $R_6$ = H, $R_4$ = $OCH_3$, n = 0)

3,46 g (20,3 mmol) 1,3-Dihydro-5-methyl-thieno(2,3)imidazol-2-thion und 3,35 g (17,3 mmol) 2-Chlormethyl-4-methoxypyridin-Hydrochlorid werden in 70 ml Ethanol suspendiert und langsam unter Rühren 18,8 ml (37,6 mmol) 2n NaOH zugetropft. Die Temperatur steigt bis auf 29° C und es bildet sich eine klare Lösung. Es wird noch 2 Stunden bei Raumtemperatur gerührt und anschließend die Lösung im Vakuum weitgehend eingedampft. Der verbleibende Rückstand wird in 70 ml Wasser aufgenommen und durch Zugabe von ca. 1 ml Eisessig ein pH-Wert von 4,5 eingestellt. Es wird viermal mit insgesamt 300 ml Chloroform ausgeschüttelt, die vereinigten organischen Phasen über Natriumsulfat getrocknet und eingedampft.

Der verbleibende ölige Rückstand wird mit Acetonitril angerieben und in der Siedehitze in 280 ml Acetonitril gelöst. Es wird nach Zusatz von Aktivkohle heiß filtriert, die Lösung auf 60 ml eingeengt, über Nacht im Kühlschrank kristallisieren gelassen, abgesaugt und mit kaltem Acetonitril gewaschen.

Ausbeute: 3,62 g hellbraune Kristalle (71,9 % d.Th.)

Fp = 173° C ($CH_3CN$)

Beispiel 3:

2-(2-Pyridyl)methylthio-3H-thieno(2,3-d)imidazol

(I: $R_1$ -$R_6$ = H, n = 0)

1,20 g (7,68 mmol) 1,3-Dihydro-thieno(2,3-d)-imidazol-2-thion (II: $R_1$ und $R_2$ = H) und 1,13 g - (6,91 mmol) 2-Chlormethylpyridin-Hydrochlorid werden in 15 ml Methanol gelöst und 7,4 ml (14,8 mmol) 2n NaOH unter Rühren innerhalb von 20 Minuten zugetropft. Es wird noch 1,5 Stunden bei Raumtemperatur gerührt und anschließend am Rotavapor eingeengt. Der Rückstand wird zwischen 60 ml Wasser und 80 ml Methylenchlorid verteilt. Zur Phasentrennung wird über ein Filtrierhilfsmittel filtriert und die wäßrige Phase noch dreimal mit je 70 ml Methylenchlorid extrahiert. Die vereinigten

organischen Phasen werden über Natriumsulfat getrocknet und eingedampft.

Das Rohprodukt (1,52 g braune Kristalle; 92,4 % d.Th.) wird aus Acetonitril unter Zusatz von Aktivkohle umkristallisiert.

Ausbeute: 1,10 g farblose Kristalle (64,3 % d.Th.)

Fp = 155 -156° C ($CH_3CN$)

Das Ausgangsmaterial kann wie folgt hergestellt werden:

1,3-Dihydro-thieno(2,3-d)imidazol-2-thion

(II: $R_1$ und $R_2$ = H)

20,0 g (0,143 mol) 1,3-Dihydro-thieno(2,3-d)-imidazol-2-on (literaturbekannt) werden bei Raumtemperatur unter mechanischem Rühren in 500 ml abs. Pyridin eingetragen. Danach werden 23,8 g - (0,107 mol) $P_2S_5$ zugegeben und das Gemisch 5,5 Stunden unter Rückfluß erhitzt. Es wird abgekühlt und das Lösungsmittel im Vakuum weitgehend abdestilliert. Der dunkle Rückstand wird in 300 ml 2n NaOH gelöst und unter heftigem Rühren in ein Gemisch aus 370 ml 2n HCl und 400 ml Ethylacetat geleert. Das zweiphasige Gemisch wird über ein Filtrierhilfsmittel abgesaugt und die Phasen getrennt. Die wäßrige Phase wird noch sechsmal mit je 150 ml Ethylacetat extrahiert, die vereinigten organischen Phasen über Natriumsulfat getrocknet und eingedampft.

Ausbeute: 8,81 g braune Kristalle (39,5 % d.Th.)

Fp = kontinuierliche Zersetzung

Beispiel 4:

2-((4-Methoxy-2-pyridyl)methylthio)-3H-thieno(2,3-d)imidazol

(I: $R_1$, $R_2$, $R_3$, $R_5$ und $R_6$ = H, $R_4$ = $OCH_3$, n = 0)

4,65 g (29,76 mmol) 1,3-Dihydro-thieno(2,3-d)-imidazol-2-thion und 4,91 g (25,30 mmol) 2-Chlormethyl-4-methoxy-pyridin-Hydrochlorid werden in 60 ml Methanol suspendiert und unter Rühren 27,6 ml (55,2 mmol) 2n KOH langsam innerhalb von 25 Minuten zugetropft. Die Temperatur steigt dabei auf 32° C. Nach beendeter Zugabe wird noch 4 Stunden bei Raumtemperatur gerührt. Anschließend wird das Reaktionsgemisch im Vakuum eingedampft, der Rückstand in 50 ml Wasser aufgenommen und durch Zutropfen von Eisessig ein pH-Wert von 4,5 eingestellt. Der ausgefallene Niederschlag wird in 80 ml Chloroform gelöst, das Gemisch heftig geschüttelt und zur Phasentrennung über ein Filtrierhilfsmittel abgesaugt. Die Phasen werden getrennt und die wäßrige noch dreimal mit je 40 ml Chloroform extrahiert. Die vereinigten organischen Phasen werden über Natriumsulfat getrocknet und eingedampft.

Das Rohprodukt (5,8 g; 82,2 % d.Th.) wird in 400 ml Acetonitril in der Siedehitze gelöst, heiß mit Aktivkohle filtriert, auf 150 ml eingeengt und auskristallisieren gelassen.

Ausbeute: 4,34 g bräunliche Kristalle (61,8 % d.Th.)

Fp = 140 -141° C($CH_3CN$)

Beispiel 5:

5-Acetyl-2-((4-methoxy-3,5-dimethyl-2-pyridyl)-methylthio)-3H-thieno(2,3-d)imidazol

(I: $R_1$ = $COCH_3$, $R_2$ und $R_6$ = H, $R_3$ und $R_5$ = $CH_3$, $R_4$ = $OCH_3$, n = 0)

5,64 g (28,4 mmol) 5-Acetyl-1,3-dihydro-thieno-(2,3-d)imidazol-2-thion und 6,00 g (27,0 mmol) 2-Chlormethyl-4-methoxy-3,5-dimethylpyridin-Hydrochloridwerden in 250 ml Methanol suspendiert und 28 ml 2n NaOH (56,0 mmol) unter Rühren innerhalb von 15 Minuten zugetropft, wobei die Temperatur bis auf 27° C ansteigt. Aus der entstehenden Lösung fällt bereits nach kurzer Zeit das Produkt aus.

Es wird insgesamt 4 Stunden bei Raumtemperatur nachgerührt und anschließend das Reaktionsgemisch weitgehend eingedampft. Der Rückstand wird in 200 ml Wasser aufgenommen und durch Zugabe von ca. 2 ml Eisessig ein pH-Wert von 4,5 eingestellt. Die Suspension wird viermal mit insgesamt 350 ml Chloroform ausgeschüttelt, die vereinigten organischen Phasen über Natriumsulfat/Aktivkohle getrocknet und eingedampft.

Das verbleibende dunkle Öl wird mit 80 ml Acetonitril kristallisiert, abgekühlt und der Niederschlag abgesaugt. Es wird dreimal mit kaltem Acetonitril

gewaschen und bei 40° C im Vakuum getrocknet.

Ausbeute: 8,92 g leicht gelbliche Kristalle (95,0 % d.Th.)

Fp = 177 -180° C ($CH_3CN$)

Das Ausgangsmaterial kann wie folgt hergestellt werden:

1-(5-Chlor-4-nitro-2-thienyl)-1-ethanon

(VIII: $R_1$ = $COCH_3$, $R_2$ = H)

In 180 ml konz. $H_2SO_4$ werden bei -20° C 30,0 g (0,1868 mol) fein zerriebenes 1-(5-Chlor-2-thienyl)-1-ethanon (VII a: $R_1$ = $COCH_3$, $R_2$ = H) unter Rühren eingetragen. Zu der rot gefärbten Lösung wird bei einer Temperatur zwischen -20° C und -25° C ein Nitriergemisch aus 7,8 ml rauchender $HNO_3$ (0,1868 mol) und 30 ml konz. $H_2SO_4$ innerhalb von 35 Minuten zugetropft.

Es wird noch 30 Minuten bei -20° C nachgerührt und anschließend das Reaktionsgemisch auf 1400 ml Eiswasser geleert. Das ausgefallene Produkt wird in 500 ml Methylenchlorid aufgenommen und die wäßrige Phase noch zweimal mit je 250 ml Methylenchlorid ausgeschüttelt. Die vereinigten organischen Phasen werden einmal mit 300 ml Wasser gewaschen, über Natriumsulfat getrocknet und eingedampft.

Das Rohprodukt (36,0 g; 93,8 % d.Th.) enthält ca. 10 % einer Verunreinigung (im DC höher laufend), die bei der weiteren Umsetzung nicht stört, jedoch durch Umkristallisieren aus Ethanol entfernt werden kann.

Ausbeute: 26,0 g leicht gelbliche Kristalle (67,7 % d.Th.)

Fp = 80° C (EtOH)

1-(5-Amino-4-nitro-2-thienyl)-1-ethanon

(IX: $R_1$ = $COCH_3$, $R_2$ = H)

In eine Lösung von 22,0 g (0,108 mol) 1-(5-Chlor-4-nitro-2-thienyl)-1-ethanon in 250 ml trockenem Dioxan wird trockenes $NH_3$-Gas eingeleitet, wobei die Temperatur bis auf 45° C ansteigt. Nach Abklingen der exothermen Reaktion wird die Temperatur durch Erwärmen auf dem Wasserbad auf 50° C gehalten und noch weitere 1,5 Stunden $NH_3$-Gas eingeleitet.

Anschließend wird von einem ausgefallenen Niederschlag abgesaugt, der verworfen wird. Das Filtrat wird bei 50° C mit Aktivkohle gerührt, erneut filtriert und eingedampft. Der verbleibende Rückstand wird aus Acetonitril umkristallisiert.

Ausbeute: 11,9 g gelbe Nadeln (59,2 % d.Th.)

Fp = 224 -225° C ($CH_3CN$)

5-Acetyl-1,3-dihydro-thieno(2,3-d)imidazol-2-thion

(II: $R_1$ = $COCH_3$, $R_2$ = H)

10,0 g (0,0537 mol) 1-(5-Amino-4-nitro-2-thienyl)-1-ethanon (IX:$R_1$ = $COCH_3$, $R_2$ = H) werden in einem Gemisch aus 150 ml Methanol und 150 ml Dioxan gelöst und mit 3 Löffeln $W_2$-Raney-Nickel als Katalysator in einer PARR-Mitteldruck-Hydrierapparatur bei Raumtemperatur bis zur berechneten Wasserstoffaufnahme in 2,5 Stunden hydriert. Der Katalysator wird über ein Filtrierhilfsmittel abgesaugt und gut mit Methanol und Dioxan gewaschen.

Das Filtrat (bestehend aus X: $R_1$ = $COCH_3$, $R_2$ = H) wird mit 8,39 g (0,0644 mol) Natriummethylxanthogenat versetzt und 4 Stunden unter Rühren und Rückfluß (Temperatur ca. 70° C) erhitzt. Nach dem Abkühlen wird weitgehend eingedampft und der Rückstand in 250 ml Wasser gelöst. Die Lösung wird 10 Minuten mit zwei Teelöffeln Aktivkohle gerührt und anschließend über ein Filtrierhilfsmittel abgesaugt. Das Filtrat wird mit konz. HCl unter Rühren langsam auf pH = 1 angesäuert, noch kurz gerührt und der ausgefallene gelbe Niederschlag abgesaugt. Die Kristalle werden 2 Stunden bei 90° C/20 mbar getrocknet.

Ausbeute: 8,2 g Kristalle (77,0 % d.Th)

Fp = 302° C (Zers.)

Beispiel 6:

2-((4-Methoxy-3,5-dimethyl-2-pyridyl)-methylthio)-3H-thieno(2,3-d)imidazol-5-carbonsäuremethylester

(I: $R_1$ = $COOCH_3$, $R_2$ und $R_6$ = H, $R_3$ = $CH_3$, $R_4$ = $OCH_3$, n = 0)

Die oben angegebene Verbindung der allgemeinen Formel I wurde analog zu der in Beispiel 5 angegebenen Arbeitsmethode durch Umsetzung von 1,3-Dihydro-thieno(2,3-d)imidazol-5-carbonsäuremethylester-2-thion mit 2-Chlormethyl-4-methoxy-3,5-dimethyl-pyridin Hydrochlorid hergestellt.

Ausbeute: 89 % d.Th.

Fp = 173 -174,5° C ($CH_3CN$)
Das Ausgangsmaterial wurde nach der in Beispiel 5 angegebenen Reaktionsfolge aus 5-Chlor-4-nitro-thiophen-2-carbonsäuremethylester (VIII: $R_1$ = $COOCH_3$, $R_2$ = H, literaturbekannt) über den 5-Amino-4-nitro-2-thiophencarbonsäuremethylester (IX: $R_1$ = $COOCH_3$, $R_2$ = H, Fp = 271° C Zers.), Reduktion zum 4,5-Diamino-2-thiophen-carbonsäuremethylester (X: $R_1$ = $COOCH_3$, $R_2$ = H) und Umsetzung dieser Verbindung mit Natriummethylxanthogenat hergestellt. Man erhält auf diese Weise das 1,3-Dihydro-thieno(2,3-d)imidazol-5-carbonsäuremethylester-2-thion (II: $R_1$ = $COOCH_3$, $R_2$ = H; Fp = 279° C, Zers.).

Beispiel 7:

5-Acetyl-2((4-methoxy-2-pyridyl)methylthio)-3H-thieno(2,3-d)-imidazol

(I: $R_1$ = $COCH_3$, $R_2$, $R_3$, $R_5$ und $R_6$ = H, $R_4$ = $OCH_3$, n =0)

1,30 g (6,567 mmol) 5-Acetyl-1,3-dihydro-thieno(2,3-d)imidazol-2-thion und 1,08 g (5,565 mmol) 2-Chlormethyl-4-methoxy-pyridin-Hydrochlorid werden in 15 ml Methanol suspendiert und unter Rühren 6,2 ml 2n NaOH (12,4 mmol) innerhalb von 10 Minuten zugetropft, wobei die Temperatur bis auf 27° C ansteigt. Es wird noch 1,5 Stunden bei Raumtemperatur gerührt.

Anschließend wird das Reaktionsgemisch weitgehend eingedampft, der Rückstand in 50 ml Wasser aufgenommen und durch Zugabe von ca. 1 ml Eisessig auf pH = 4 gebracht. Es wird dreimal mit je 40 ml Chloroform ausgeschüttelt, die vereinigten organischen Phasen über Natriumsulfat getrocknet und eingedampft.

Das Rohprodukt (1,52 g braunes Öl) wird mit Acetonitril angerieben und aus Acetonitril unter Zusatz von Aktivkohle umkristallisiert.

Ausbeute: 0,76 g farblose Kristalle (42,8 % d.Th.)

Fp = 192 -194° C ($CH_3CN$)

Beispiel 8:

5-Acetyl-2-(2-pyridyl)methylthio-3H-thieno(2,3-d)-imidazol

(I: $R_1$ = $COCH_3$, $R_2$ -$R_6$ = H, n = 0)

1,50 g (7,565 mmol) 5-Acetyl-1,3-dihydro-thieno(2,3-d)imidazol-2-thion und 1,12 g (6,809 mmol) 2-Chlormethylpyridin-Hydrochlorid werden in 60 ml Methanol suspendiert und 7,2 ml 2n NaOH (14,4 mmol) unter Rühren innerhalb von 8 Minuten zugetropft, wobei die Temperatur bis auf 26° C ansteigt. Es wird noch 2 Stunden bei Raumtemperatur gerührt.

Die entstandene klare Lösung wird weitgehend eingedampft, der Rückstand in 80 ml Wasser aufgenommen und durch Zugabe von 1,2 ml Eisessig ein pH-Wert von 4,5 eingestellt. Es wird dreimal mit insgesamt 150 ml Chloroform extrahiert, die organische Phase über Natriumsulfat getrocknet und eingedampft. Die verbleibenden Kristalle werden aus Acetonitril umkristallisiert.

Ausbeute: 1,85 g farblose Kristalle (84,5 % d.Th.)
Fp=171 -173° C ($CH_3CN$)

Beispiel 9:

5-Methyl-2-(2-pyridyl)-methylsulfinyl-3H-thieno(2,3-d)imidazol

(I: $R_1$ = $CH_3$, $R_2$ -$R_6$ = H, n = 1)

1,0 g (3,826 mmol) 5-Methyl-1-(2-pyridyl)-methylthio-3H-thieno(2,3-d)imidazol wird in 20 ml Chloroform bei Raumtemperatur gelöst und die Lösung auf -10° C gekühlt. Bei einer Temperatur zwischen -12° C und -9° C wird eine Lösung von 0,76 g (3,749 mmol) 85 proz. 3-Chlorperbenzoesäure in 10 ml Chloroform in 10 Minuten unter Rühren zugetropft. Nach beendeter Zugabe wird noch 10 Minuten bei -10° C gerührt.

Das Reaktionsgemisch wird zweimal mit je 10 ml ges. Natriumhydrogencarbonatlösung ausgeschüttelt und die vereinigten organischen Phasen über Natriumsulfat getrocknet und eingedampft.

Das Rohprodukt (0,96 g; 90,5 % d.Th.) wird aus Acetonitril umkristallisiert.

Ausbeute: 0,76 g farblose Kristalle (71,6 % d.Th.)

Fp = 175 -176° C Zers. ($CH_3CN$)

Beispiel 10:

5-Methyl-2-((4-methoxy-2-pyridyl)methylsulfinyl)-3H-thieno-(2,3-d)imidazol

(I: $R_1$ = $CH_3$, $R_2$, $R_3$, $R_5$ und $R_6$ = H, $R_4$ = $OCH_3$, n = 1)

6,0 g (20,59 mmol) 5-Methyl-2-((4-methoxy-2-pyridyl)methylthio)-3H-thieno(2,3-d)imidazol werden in 120 ml Chloroform bei Raumtemperatur gelöst. Die Lösung wird auf -12° C gekühlt und innerhalb von 20 Minuten eine Lösung von 4,10 g (20,18 mmol) 85 proz. 3-Chlorperbenzoesäure in 60 ml Chloroform bei einer Temperatur zwischen -12° C und -8° C unter Rühren zugetropft. Nach beendeter Zugabe wird noch 10 Minuten bei -10° C gerührt.

Anschließend wird zweimal mit je 25 ml ges. Natriumhydrogencarbonatlösung extrahiert, die wäßrigen Phasen zweimal mit wenig Chloroform gewaschen, die vereinigten organischen Phasen über Natriumsulfat getrocknet und eingedampft.

Der verbleibende Rückstand (ca. 6 g rötliches Öl) wird mit wenig Acetonitril kristallisiert und in 40 ml DMF bei 80° C gelöst. Es wird heiß mit Aktivkohle filtriert, in 220 ml 60° C warmes Acetonitril geleert und langsam abkühlen gelassen. Die Kristallisation wird über Nacht im Kühlschrank vervollständigt.

Danach wird abgesaugt und mit kaltem Acetonitril gewaschen.

Ausbeute: 4,81 g farblose Kristalle (76 % d.Th.)

Fp = 183 -184° C (DMF/$CH_3CN$)

Beispiel 11:

2-(2-Pyridyl)methylsulfinyl-3H-thieno(2,3-d)imidazol

(I: $R_1$ -$R_6$ = H, n = 1)

0,45 g (1,82 mmol) 2-(2-Pyridyl)methylthio-3H-thieno(2,3-d)imidazol werden in 10 ml Chloroform gelöst und bei einer Temperatur zwischen -11° und -6° C eine Lösung von 0,37 g (1,82 mmol) 85 proz. 3-Chlorperbenzoesäure in 5 ml Chloroform unter Rühren zugetropft. Es wird noch 30 Minuten bei einer Temperatur unter 0° C gerührt, die Lösung mit wenig Methylenchlorid verdünnt und zweimal mit je 5 ml ges. Natriumhydrogencarbonatlösung gewaschen.

Die wäßrige Phase wird zweimal mit je 5 ml Methylenchlorid extrahiert, die vereinigten organischen Phasen über Natriumsulfat getrocknet und eingedampft.

Der nach Anreiben mit Acetonitril kristalline Rückstand (0,42 g; 87,7 % d.Th.) wird aus Acetonitril/Aktivkohle umkristallisiert.

Ausbeute: 0,33 g farblose Kristalle (68,9 % d.Th.)

Fp = 151 -152° C Zers. ($CH_3CN$)

Beispiel 12:

2-((4-Methoxy-2-pyridyl)methylsulfinyl)-3H-thieno-(2,3-d)imidazol

(I: $R_1$ -$R_3$, $R_5$ und $R_6$ = H, $R_4$ = $OCH_3$, n = 1)

4,30 g (15,50 mmol) 2-((4-Methoxy-2-pyridyl)-methylthio)-3H-thieno-(2,3-d)imidazol werden in 90 ml Chloroform bei Raumtemperatur gelöst und die Lösung auf -10° C gekühlt. Danach wird eine Lösung von 3,08 g (15,19 mmol) 85 proz. 3-Chlorperbenzoesäure in 35 ml Chloroform unter Rühren bei einer Temperatur zwischen -11° und -8° C innerhalb von 30 Minuten zugetropft. Anschließend wird noch 15 Minuten bei -10° C gerührt.

Die Lösung wird zweimal mit insgesamt 30 ml ges. Natriumhydrogencarbonatlösung extrahiert, die wäßrige Phase dreimal mit je 20 ml Chloroform ausgeschüttelt und die vereinigten organischen Phasen über Natriumsulfat getrocknet und eingedampft.

Das Rohprodukt (5,2 g rotes Öl) wird mit wenig Acetonitril angerieben und die bräunlichen Kristalle in 22 ml DMF bei 80° C gelöst. Es wird mit

Aktivkohle versetzt, heiß filtriert und in 130 ml 60° C warmes Acetonitril geleert. Es wird langsam abkühlen gelassen und die Kristallisation im Kühlschrank über Nacht vervollständigt.

Ausbeute: 4,03 g farblose Kristalle (88,6 % d.Th.)

Fp = 157 -9° C Zers. ($CH_3CN$)

Beispiel 13:

5-Acetyl-2-((4-methoxy-3,5-dimethyl-2-pyridyl)-methylsulfinyl)-3H-thieno(2,3-d)imidazol

(I: $R_1$ = $COCH_3$, $R_2$ und $R_6$ = H, $R_3$ und $R_5$ = $CH_3$, $R_4$ = $OCH_3$, n = 1)

4,5 g (12,95 mmol) 5-Acetyl-2-(4-methoxy-3,5-dimethyl-2-pyridyl)-methylthio)-3H-thieno(2,3-d)-imidazol werden in 120 ml Chloroform bei Raumtemperatur fast vollständig gelöst und die Lösung auf -10° C gekühlt. Bei einer Temperatur zwischen -8° C und -10° C wird eine Lösung von 2,58 g - (12,69 mmol) 85 proz. 3-Chlorperbenzoesäure in 40 ml Chloroform innerhalb von 25 Minuten unter Rühren zugetropft.

Es wird noch 10 Minuten bei -10° C gerührt und die nun völlig klare Lösung dreimal mit insgesamt 60 ml ges. Natriumhydrogencarbonatlösung extrahiert. Die wäßrige Phase wird dreimal mit wenig Chloroform gewaschen, die vereinigten organischen Phasen über Natriumsulfat getrocknet und eingedampft.

Der verbleibende Rückstand (ca. 5 g rotes Öl) wird in 50 ml DMF bei 80° C gelöst und mit Aktivkohle filtriert. Die Lösung wird in 300 ml 60° C warmes Acetonitril geleert, langsam abkühlen gelassen und die Kristallisation über Nacht im Kühlschrank vervollständigt. Es wird abgesaugt und dreimal mit Acetonitril gewaschen.

Ausbeute: 3,30 g farblose Kristalle (70,1 % d.Th.)

Fp = 190 -191° C (DMF/$CH_3CN$)

Beispiel 14:

5-Acetyl-2-((4-methoxy-2-pyridyl)methylsulfinyl)-3H-thieno-(2,3-d)imidazol

(I: $R_1$ = $COCH_3$, $R_2$, $R_3$, $R_5$ und $R_6$ = H, $R_4$ = $OCH_3$, n = 1)

0,53 g (1,628 mmol) 5-Acetyl-2-(4-methoxy-2-pyridyl)methylthio)-3H-thieno(2,3-d)imidazol werden in 15 ml Chloroform suspendiert und bei einer Temperatur zwischen -12° und -8° C eine Lösung aus 0,33 g (1,628 mmol) 85 proz. 3-Chlorperbenzoesäure in 6 ml Chloroform unter Rühren in 7 Minuten zugetropft, wobei eine klare Lösung entsteht. Es wird noch 10 Minuten bei minus 10° C gerührt. Anschließend wird zweimal mit je 6 ml ges. Natriumhydrogencarbonatlösung extrahiert, die wäßrigen Phasen zweimal mit je 5 ml Chloroform rückgeschüttelt, die vereinigten organischen Phasen über Natriumsulfat getrocknet und eingedampft. Das ölige Rohprodukt wird aus Acetonitril/Aktivkohle umkristallisiert.

Ausbeute: 0,37 g farblose Kristalle (67,8 % d.Th.)

Fp = 159 -162° C Zers. ($CH_3CN$)

Beispiel 15:

5-Acetyl-2-((4-methoxy-2-pyridyl)methylsulfinyl)-3H-thieno(2,3-d)imidazol

(I: $R_1$ = $COCH_3$, $R_2$, $R_3$, $R_5$ und $R_6$ = H, $R_4$ = $OCH_3$, n = 1)

0,53 g (1,628 mmol) 5-Acetyl-2-(4-methoxy-2-pyridyl)methylthio)-3H-thieno(2,3-d)imidazol werden in 15 ml Eisessig aufgenommen und bei 5 bis 10°C mit einer Lösung von 181 mg (1,628 mmol) 30 proz. $H_2O_2$ langsam versetzt, wobei eine klare Lösung entsteht. Es wird noch 20 Minuten bei Raumtemperatur gerührt. Anschließend wird mit 100 ml Wasser verdünnt, dreimal mit je 20 ml Chloroform ausgeschüttelt und die vereinigten organischen Phasen mit Natriumhydrogencarbonatlösung neutral gewaschen, über Natriumsulfat getrocknet und eingeengt. Das ölige Rohprodukt wird aus Acetonitril/Aktivkohle umkristallisiert.

Ausbeute: 0,28 g farblose Kristalle (51,3 % d.Th.)

Fp = 159 -162° C Zers. ($CH_3CN$)

Beispiel 16:

5-Acetyl-2-(2-pyridyl)methylsulfinyl-3H-thieno(2,3-d)imidazol

(I: $R_1$ = $COCH_3$, $R_2$ -$R_6$ = H, n = 1)

1,0 g (3,46 mmol) 5-Acetyl-2-(2-pyridyl)-methylthio-3H-thieno-(2,3-d)imidazol wird bei Raumtemperatur in 15 ml Chloroform fast vollständig gelöst und die Lösung auf -12° C gekühlt. Bei einer Temperatur zwischen -14° und -9° C wird eine Lösung von 0,69 g (3,39 mmol) 85 proz. 3-Chlorperbenzoesäure in 10 ml Chloroform innerhalb von 10 Minuten zugetropft.

Es wird noch 10 Minuten bei -10° C gerührt, die mittlerweile völlig klare Lösung mit wenig Chloroform verdünnt und zweimal mit je 7 ml gesättigter Natriumhydrogencarbonatlösung extrahiert. Die organische Phase wird über Natriumsulfat getrocknet und eingedampft. Der verbleibende Rückstand wird mit Acetonitril kristallisiert und aus Acetonitril unter Zusatz von Aktivkohle umkristallisiert.

Ausbeute: 0,78 g farblose Kristalle (73,9 % d.Th.)

Fp = 194 -197° C ($CH_3CN$)

Beispiel 17:

2-((4-Methoxy-3,5-dimethyl-2-pyridyl)-methylsulfinyl)-3H-thieno(2,3-d)imidazol-5-carbonsäuremethylester

(I: $R_1$ = $COOCH_3$, $R_2$ und $R_6$ = H, $R_3$ und $R_4$ = $OCH_3$, n = 1)

Die oben angegebene Verbindung der allgemeinen Formel I wird analog zu der in Beispiel 16 angegebenen Arbeitsweise durch partielle Oxidation von 2-((4-Methoxy-3,5-dimethyl-2-pyridyl)-methylthio)3H-thieno(2,3-d)imidazol-5-carbonsäuremethylether mit Perbenzoesäure in Methylenchlorid als Lösungsmittel erhalten.

Ausbeute: 72,5 % d.Th.

Fp = 179 -179,5° C ($CH_3CN$)

**Ansprüche**

1. Derivate von Thieno(2,3-d)imidazolen der allgemeinen Formel I des Formelblattes,

in der

$R_1$ Wasserstoff, niederes Alkyl, Chlor, Brom, Acetyl, Cyclopropylcarbonyl, Methoxycarbonyl oder Ethoxycarbonyl,

$R_2$ Wasserstoff oder niederes Alkyl,

$R_3$, $R_5$ und $R_6$ unabhängig voneinander Wasserstoff oder niederes Alkyl,

$R_4$ Wasserstoff oder niederes Alkoxy und

n 0 oder 1 bedeuten,

und deren pharmazeutisch verträgliche Säureadditionssalze.

2. Verbindungen der in Anspruch 1 definierten allgemeinen Formel I, worin n die Zahl 1 bedeutet.

3. Verbindungen der allgemeinen Formel I nach einem der Ansprüche 1 oder 2,

worin

$R_1$ Wasserstoff, Acetyl, Methoxycarbonyl, Ethoxycarbonyl,

$R_2$ Wasserstoff, $R_3$, $R_5$ und $R_6$ Wasserstoff oder Methyl und

$R_4$ Wasserstoff oder Methoxy bedeuten.

4. Verfahren zur Herstellung von Verbindungen der in Anspruch 1 definierten allgemeinen Formel I, dadurch gekennzeichnet, daß man

a) eine Verbindung der allgemeinen Formel II des Formelblattes,

worin $R_1$ und $R_2$ die in Formel I angegebene Bedeutung haben mit einer Verbindung der allgemeinen Formel III des Formelblattes,

worin

X für Chlor oder Brom steht und

$R_3$, $R_4$, $R_5$ und $R_6$ die bei Formel I angegebene Bedeutung haben, in Gegenwart von mindestens 2 Äquivalenten einer starken Base umsetzt, worauf man

b) gegebenenfalls die so erhaltenen Verbindungen der allgemeinen Formel I, worin n = 0 bedeutet, mit äquivalenten Mengen einer organischen Persäure oder Wasserstoffperoxid zu einer Verbindung der allgemeinen Formel I umsetzt, in der n die Zahl 1 bedeutet, und

c) erwünschtenfalls eine erhaltene Verbindung der allgemeinen Formel I in ein pharmazeutisch verträgliches Säureadditionssalz überführt.

5. Pharmazeutische Präparate, enthaltend Verbindungen der allgemeinen Formel I nach Anspruch 1 oder deren Säureadditionssalze in Kombination mit üblichen Hilfs-und Trägestoffen.

6. Verbindungen nach Anspruch 1 zur Verwendung als Wirkstoffe für Arzneimittel zur Behandlung und Prophylaxe von Krankheiten, die durch erhöhte Magensekretion hervorgerufen werden, wie Magen-und Zwölffingerdarmgeschwüren.

Patentansprüche für den Vertragsstaat : AT

1. Verfahren zur Herstellung neuer Derivate von Thieno(2,3-d)imidazolen der allgemeinen Formel des Formelblattes,

in der

$R_1$ Wasserstoff, niederes Alkyl, Chlor, Brom, Acetyl, Cyclopropylcarbonyl, Methoxycarbonyl oder Ethoxycarbonyl,

$R_2$ Wasserstoff oder niederes Alkyl,

$R_3$, $R_5$ und $R_6$ unabhängig voneinander Wasserstoff oder niederes Alkyl,

$R_4$ Wasserstoff oder niederes Alkoxy und

n O oder 1 bedeuten,

und von deren pharmazeutisch verträglichen Säureadditionssalzen, dadurch gekennzeichnet, daß man

a) eine Verbindung der allgemeinen Formel II des Formelblattes,

worin $R_1$ und $R_2$ die in Formel I angegebene Bedeutung haben mit einer Verbindung der allgemeinen Formel III des Formelblattes,

worin

X für Chlor oder Brom steht und

$R_3$, $R_4$, $R_5$ und $R_6$ die bei Formel I angegebene Bedeutung haben, in Gegenwart von mindestens 2 Äquivalenten einer starken Base umsetzt, worauf man

b) gegebenfalls die so erhaltenen Verbindungen der allgemeinen Formel I, worin n = 0 bedeutet, mit äquivalenten Mengen einer organischen Persäure oder Wasserstoffperoxid zu einer Verbindung der allgemeinen Formel I umsetzt, in der n die Zahl 1 bedeutet, und

c) erwünschtenfalls eine in Stufe a) oder b) erhaltene Verbindung der allgemeinen Formel I in ein pharmazeutisch verträgliches Säureadditionssalz überführt.

2. Verfahren nach Anspruch 1, dadurch gekennzeichnet, daß Verbindungen der allgemeinen Formel I hergestellt werden, in der n die Zahl 1 bedeutet.

3. Verfahren nach einem der Ansprüche 1 oder 2, dadurch gekennzeichnet, daß man eine Verbindung der allgemeinen Formel II, worin $R_1$ Wasserstoff, Acetyl, Methoxycarbonyl oder Ethoxycarbonyl und $R_2$ Wasserstoff bedeuten, mit einer Verbindung der allgemeinen Formel III, worin $R_3$, $R_5$ und $R_6$ Wasserstoff oder Methyl und $R_4$ Wasserstoff oder Methoxy bedeuten, umsetzt.

4. Verfahren nach einem der Ansprüche 1 bis 3, dadurch gekennzeichnet, daß man die Umsetzung in Stufe a) in einem niedrigsiedendem Alkohol, vorzugsweise Methanol, durchführt.

5. Verfahren nach einem der Ansprüche 1 bis 4, dadurch gekennzeichnet, daß man in Stufe a) die Verbindung der allgemeinen Formel II im Überschuß einsetzt.

6. Verfahren nach einem der Ansprüche 1 bis 5, dadurch gekennzeichnet, daß man die Oxidation in Stufe b) mit äquivalenten Mengen 3-Chlorperbenzoesäure, Perbenzoesäure oder Peressigsäure durchführt.

7. Verfahren nach Anspruch 6, dadurch gekennzeichnet, daß man die Oxidation in Stufe b) in Methylenchlorid oder Chloroform bei -5°C bis -50°C durchführt.

8. Verfahren nach einem der Ansprüche 1 bis 5, dadurch gekennzeichnet, daß man die Oxidation in Stufe b) mit äquivalenten Mengen Wasserstoffperoxid durchführt.

9. Verfahren nach Anspruch 8, dadurch gekennzeichnet, daß man die Oxidation in Stufe b) mit 30 %-igem Wasserstoffperoxid in Eisessig durchführt.

10. Verfahren zur Herstellung von pharmazeutischen Präparaten, dadurch gekennzeichnet, daß man Verbindungen der allgemeinen Formel I bzw. deren Salze mit einem für die orale oder parenterale Applikation geeigneten, pharmazeutischen, organischen oder anorganischen Trägermaterial zu festen oder flüssigen Arzneiformen verarbeitet.

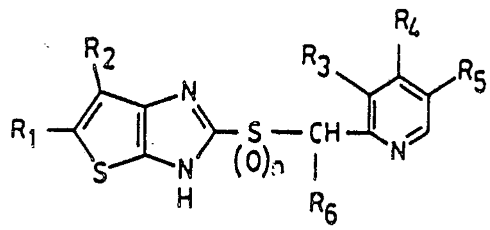

I

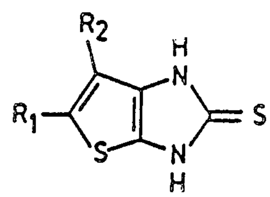

II

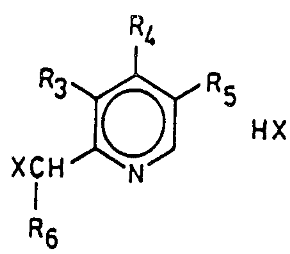

III